# EUROPEAN PATENT APPLICATION

(11) **EP 1 518 528 A1**
(43) Date of publication of application: **30.03.2005**
(21) Application number: 04002220.4
(22) Date of filing: 02.02.2004
(51) Int. Cl.: A61F 13/84, A61F 15/00

(54) **Package for disposable tissues and liquid**

(30) Priority: 26.09.2003 KR 2003067099
(71) Applicant: Cho, Kwang-Rae, 618-807 Busan (KR)
(72) Inventor: Cho, Kwang-Rae, 618-807 Busan (KR)
(74) Representative: TER MEER STEINMEISTER & PARTNER GbR

(57) **Abstract**

The present invention relates to a disposable tissue structure which includes an upper unit having a plurality of compressed tissue storing units, each compressed tissue storing unit having a lower opened side and an upper closed side and being hollow and upwardly protruded from an upper surface of the upper unit for storing a compressed tissue therein which is compressed using a nonwoven fabric, pulp or the like and is formed in a certain shape, so that the compressed tissue is changed to a disposable wet tissue when a certain liquid is absorbed to the compressed tissue, a middle unit which is sealingly adhered to a lower surface of the upper unit and has a certain thickness and tearing strength, the middle unit being forked in a flat plate shape, and a lower unit which is sealingly adhered to a lower surface of the middle unit and has a plurality of liquid storing units which are formed in the same construction as the inverted upper unit, the liquid storing unit having an opened upper side and closed lower side and being hollow and downwardly protruded from a lower surface of the lower unit for thereby storing a liquid having a certain function.

## Description

### BACKGROUND OF THE INVENTION

### 1. Field of the Invention

The present invention relates to a disposable tissue structure, and an improved disposable structure which is capable of instantly fabricating a disposable wet tissue in such a manner that a compressed dry tissue fabricated by rolling and compressing a certain shaped dry non-woven fabric absorbs an integrally provided liquid for thereby changing a compressed dry tissue to a disposable wet tissue without using a separately provided liquid compared to a conventional art, so that a user can use a disposable wet tissue anytime and anywhere, and a disposable wet tissue is naturally rotten and decomposed without producing an environmental problem, thus implementing an environment friendly tissue.

### 2. Description of the Background Art

As an industry is advanced, and due to its convenience, the use of a disposable tissue is gradually increased. Recently, the use of a compressed tissue(hereinafter referred to as CT) is sharply increased based on its small volume and certain shaped structure.

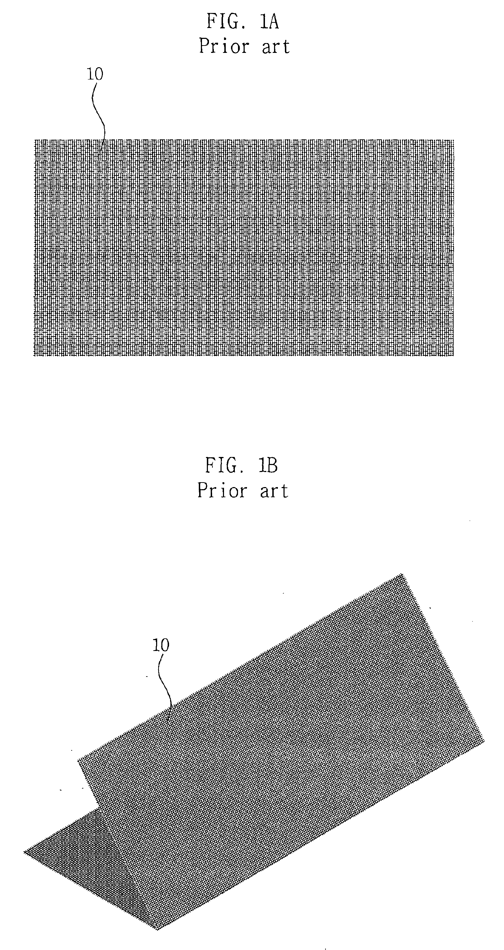

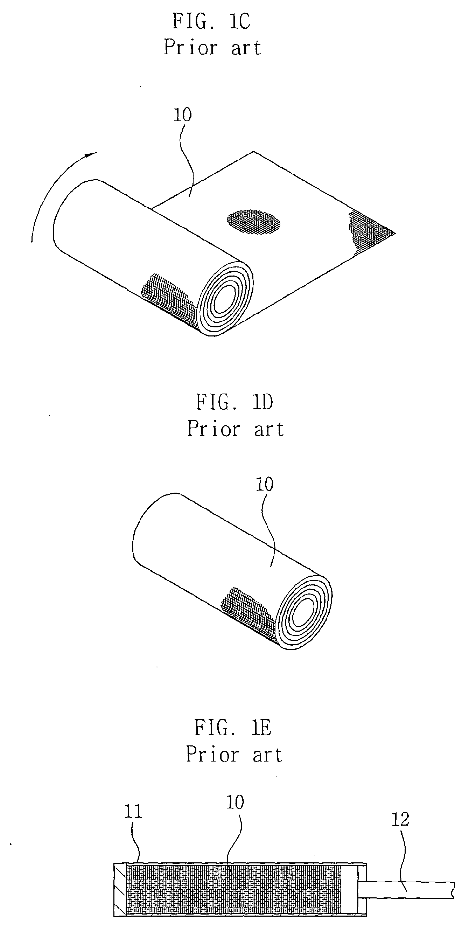

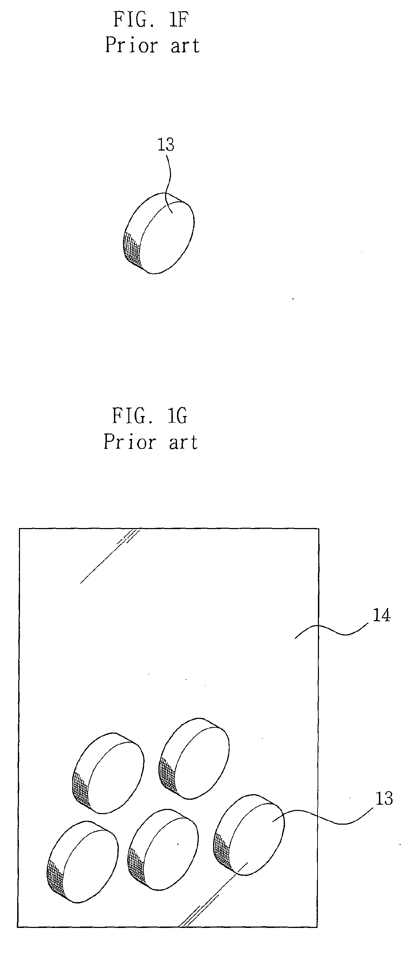

Referring to Figures 1A through 1G, the fabrication of the disposable compressed tissue of the conventional art will be described.

First, as shown in Figure 1A, a paper, pulp, or preferably a non-woven fabric 10 having a certain side like a handkerchief is provided. As shown in Figure 1B, the thusly prepared handkerchief sized non-woven fabric 10 is folded by a certain number in a certain direction, preferably, in a longitudinal direction. As shown in Figures 1C and 1D, the folded non-woven fabric 10 is rolled in a certain direction for thereby fabricating a cylindrically rolled non-woven fabric 10. The cylindrical non-woven fabric 10 is inserted into the interior of a cylinder 11 and is compressed using a piston 12 for thereby fabricating a compressed and size-decreased non-woven fabric 10 which is very hard, so that the compressed tissue 13 of Figure 1 F is fabricated. At this time, the compressed tissue 13 is dry and very hard like stone. The thusly fabricated compressed tissues 13 are stored into the interior of a vinyl pack 14.

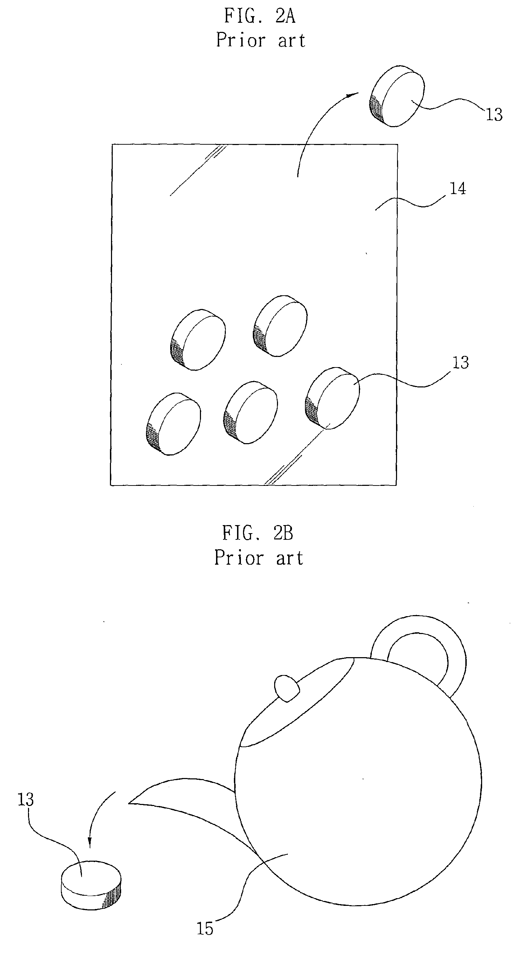

Referring to Figures 2A through 2D, the operation of the conventional compressed tissue will be briefly described.

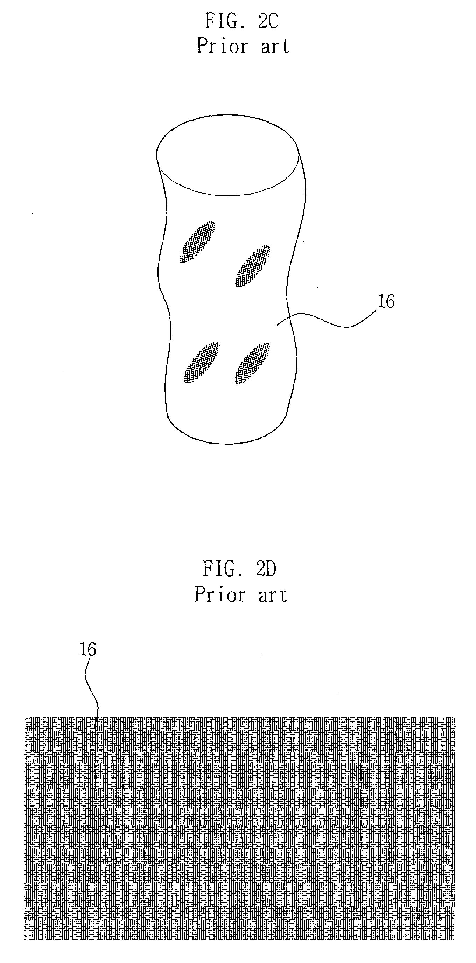

As shown in Figure 2A, a plurality of compressed tissues 13 are stored in the vinyl pack 14. When a user wants to use the compressed tissue 13, the user takes out one compressed tissue 13 stored in the vinyl pack 14. A liquid, preferably, water stored in a kettle 15 which is provided separately from the compressed tissue 13 is added to the compressed tissue 13 as shown in Figure 2B. When the compressed dry tissue 13 absorbs liquid in the above manner, the compressed tissue 13 is recovered to the wet tissue 13 which has a certain shape before the tissue 13 is not compressed, for thereby fabricating a cylindrical wet tissue 16 as shown in Figure 2C. The thusly fabricated cylindrical wet tissue 16 is unfolded, so that it is possible to fabricate the wet tissue 16 as shown in Figure 2D. At this time, the thusly fabricated wet tissue 16 has the same construction as the non-woven fabric 10 before the non-woven fabric 10 is rolled and compressed, but the thusly fabricated tissue 16 is wet. Therefore, the above wet tissue may be used like the commonly used wet tissue.

However, there are the following problems in the conventional compressed tissue even though its convenience.

First, in order to use the dry compressed tissue, a certain liquid like water must be externally provided. Therefore, in the conventional art, water must be provided in a separate container in order to use the conventional compressed tissue or a user must carry a certain liquid or water separately from the compressed tissue or must move to a certain place where water is provided. There are much inconvenience for using the conventional compressed tissue.

Second, since the conventional dry compressed tissue is stored in a certain simple vinyl pack without a certain function, an external appearance of the vinyl with compressed tissues is bad for women to use for a portable item.

Third, since the conventional dry compressed tissues are stored in a simple vinyl pack, etc. when a user takes out one among a plurality of the compressed dry tissues, the user may touch other compressed tissues for thereby polluting other compressed tissue by his hand, so that it is impossible to implement a sanitary use of the compressed tissue.

Fourth, the vinyl pack with compressed tissues is opened whenever the compressed tissue is used, so that a certain foreign pollutant may be penetrated into the vinyl pack through an opened side, whereby it is impossible to implement a sanitary use of the compressed tissue.

Fifth, when a user wants to use the compressed tissue, the compressed must absorb a certain liquid like water. At this time, if the compressed tissue absorbs water over a certain amount, the wet tissue having too water is fabricated, so that it is difficult to use the same. If the compressed tissue absorbs less amount of liquid, it is impossible to implement a wet tissue having a proper amount of water.

### SUMMARY OF THE INVENTION

Accordingly, it is a first object of the present invention to provide a disposable tissue structure which is capable of instantly fabricating a wet tissue and conveniently using the same without separately providing a certain liquid by providing a compressed tissue storing unit adapted to store a compressed tissue and a liquid storing unit adapted to store a certain liquid which are provided in an integral structure.

It is a second object of the present invention to provide a disposable tissue structure which is capable of using a sanitary compressed tissue without any pollutant by providing a plurality of sealed compressed tissue storing units which are each adapted to individually store the compressed tissue based on a sealed method.

It is a third object of the present invention to provide a disposable tissue structure which is easily used by users without distinction of age and sex by sealing-separating a compressed tissue and a liquid and providing a middle layer unit which is torn by a certain force.

It is a fourth object of the present invention to provide a disposable tissue structure which is well adapted for fabricating a wet tissue having a certain function in such a manner that a liquid having a certain function such as a sterile function, sterilization function, deodorization function, skin caring function, aging prevention function, aroma providing function, etc. is stored in a liquid storing unit which is sealingly formed below a compressed tissue.

It is a fifth object of the present invention to provide a disposable tissue structure which is capable of instantly fabricating a sanitary wet tissue which may be stored for a long time without deterioration by storing a fully dry compressed tissue in a compressed tissue storing unit and a fully sterilized liquid in a liquid storing unit.

It is a sixth object of the present invention to provide a disposable tissue structure which is capable of implementing an optimum wet tissue by fabricating a wet tissue in such a manner that a most preferred amount of liquid is added to a compressed tissue by providing a liquid storing unit which has a proper volume determined so that a user can use comfortably.

It is a seventh object of the present invention to provide a disposable tissue structure which is capable of implementing a beautiful outer appearance of a product in such a manner that the disposable compressed structure is fabricated in various colors and shapes for example an elliptical shape, rectangular shape, circular shape, etc.

It is an eighth object of the present invention to provide a disposable tissue structure of which the weight is gradually decreased as the disposable tissue is used in such a manner that the compressed tissue storing unit which is used is cut and discarded by forming a straight line cut portion between each compressed tissue storing unit, thus implementing a carry easy product.

To achieve the above objects, there is provided a disposable tissue structure according to a first embodiment of the present invention which includes an upper unit having a plurality of compressed tissue storing units, each compressed tissue storing unit having a lower opened side and an upper closed side and being hollow and upwardly protruded from an upper surface of the upper unit for storing a compressed tissue therein which is compressed using a non-woven fabric, pulp or the like and is formed in a certain shape, so that the compressed tissue is changed to a disposable wet tissue when a certain liquid is absorbed to the compressed tissue, a middle unit which is sealingly adhered to a lower surface of the upper unit and has a certain thickness and tearing strength, the middle unit being forked in a flat plate shape, and a lower unit which is sealingly adhered to a lower surface of the middle unit and has a plurality of liquid storing units which are formed in the same construction as the inverted upper unit, the liquid storing unit having an opened upper side and closed lower side and being hollow and downwardly protruded from a lower surface of the lower unit for thereby storing a liquid having a certain function.

To achieve the above objects, there is provided a disposable tissue structure according to a second embodiment of the present invention which includes a flat plate shaped upper unit which has a certain thickness and a tearing strength, and a lower unit which is sealingly adhered to a lower surface of the upper unit and has a plurality of tissue and liquid storing units, each of the tissue and liquid storing units having an opened upper side and a closed lower side and being downwardly protruded in a certain shape for storing a compressed tissue therein which is compressed using a non-woven fabric, pulp or the like and is formed in a certain shape, so that the compressed tissue is changed to a disposable wet tissue when a certain liquid is absorbed to the compressed tissue, and each of the tissue and liquid storing units being capable of storing a certain liquid therein for thereby fabricating a disposable wet tissue using a compressed tissue.

### BRIEF DESCRIPTION OF THE DRAWINGS

The present invention will become better understood with reference to the accompanying drawings which are given only by way of illustration and thus are not limitative of the present invention, wherein;
Figures 1A through 1G are schematic views illustrating a process for fabricating a conventional compressed tissue, of which:
Figure 1A is a view illustrating a non-woven fabric which before the non-woven fabric is folded in the conventional art;
Figure 1B is a view illustrating a state that the non-woven fabric of Figure 1A is folded in a longitudinal direction one time in the conventional art;
Figure 1C is a view illustrating a state that the non-woven fabric folded by a certain number like Figure 1B is rolled in a cylindrical shape in the conventional art;
Figure 1D is a view illustrating a state that the non-woven fabric of Figure 1A is finally rolled in a cylindrical shape in the conventional art;
Figure 1E is a view illustrating a state that a cylindrical non-woven fabric is inserted into a certain shaped cylinder and is compressed by a piston for thereby fabricating a compressed tissue in the conventional art;
Figure 1F is a perspective view illustrating the construction of a compressed tissue in the conventional art; and
Figure 1G is a perspective view illustrating a state that a compressed tissue is stored in a vinyl pack for use of the conventional compressed tissue;
Figures 2A through 2D are views illustrating a process for using a conventional compressed tissue, of which:
Figure 2A is a view illustrating a state that one compressed tissue is picked out from a vinyl pack used for storing a plurality of compressed tissues in the conventional art;
Figure 2B is a view illustrating a procedure that a certain liquid, preferably, water is provided to a compressed tissue for recovering the compressed tissue to a non-compressed state tissue for using a conventional compressed tissue;
Figure 2C is a view illustrating a cylindrical wet tissue which is recovered to a state that the compressed is not performed by adding a certain liquid to a conventional compressed tissue; and
Figure 2D is a view illustrating a non-folded wet tissue for using a conventional cylindrical wet tissue;

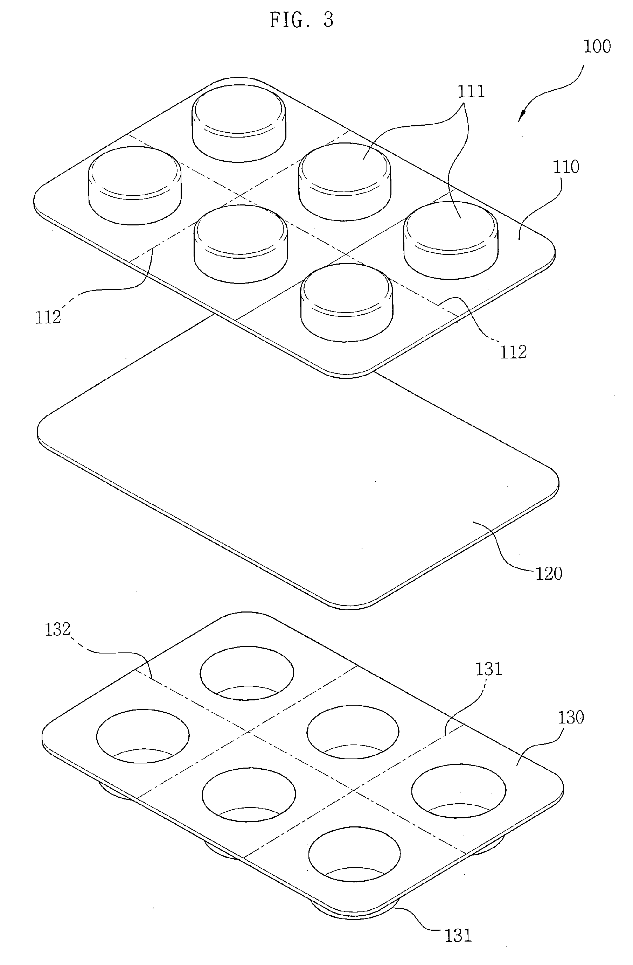

Figure 3 is a disassembled perspective view illustrating a state that a disposable tissue structure is disassembled according to a first embodiment of the present invention;

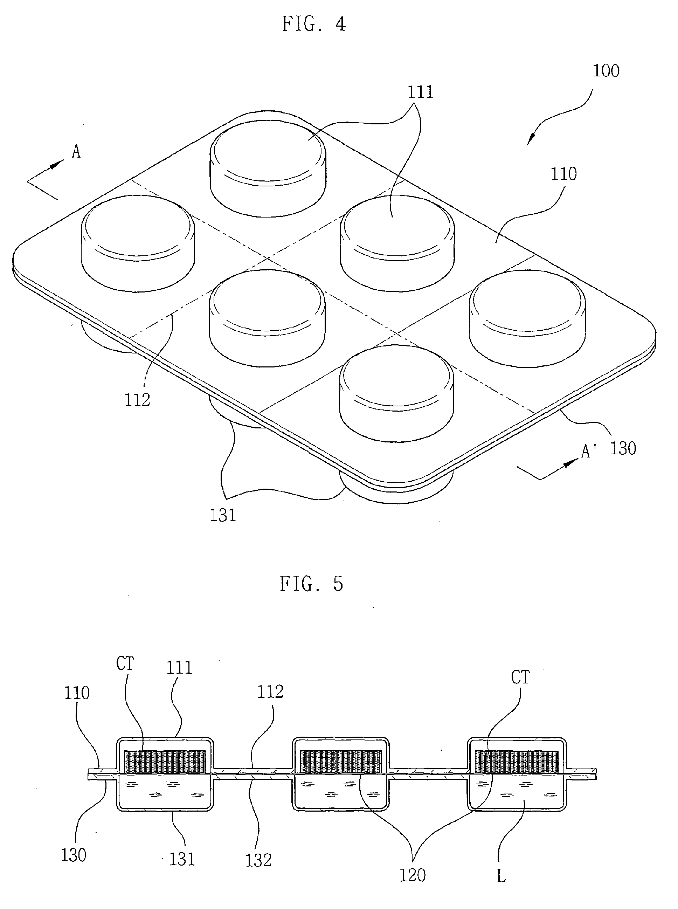

Figure 4 is a perspective view illustrating the construction of a disposable tissue structure according to a first embodiment of the present invention;
Figure 5 is a cross sectional view taken along line A-A' of Figure 4 for describing a cross section state of a disposable tissue structure according to a first embodiment of the present invention;

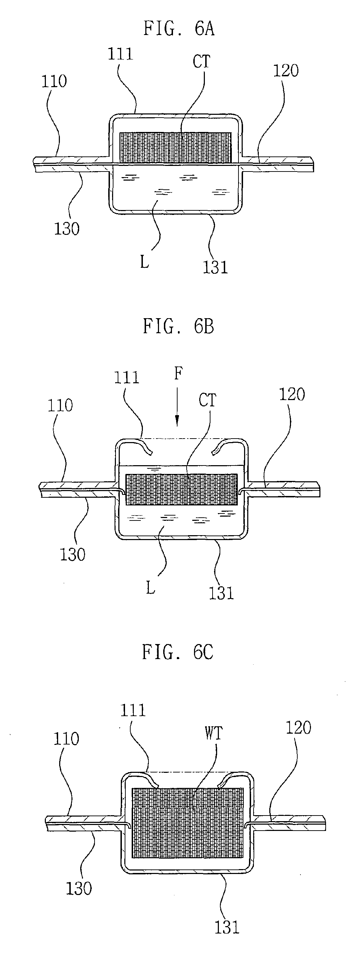

Figures 6A through 6F are cross sectional views for describing an operation of a disposable tissue structure according to a first embodiment of the present invention, of which:
Figure 6A is a cross sectional view illustrating a state that a compressed tissue and a liquid are stored in a compressed tissue storing unit and a liquid storing unit according to a first embodiment of the present invention;
Figure 6B is a cross sectional view illustrating a state that a certain force F is applied from an upper side of a compressed tissue storing unit of a disposable tissue structure according to a first embodiment of the present invention;
Figure 6C is a cross sectional view illustrating a process that a compressed tissue of a disposable tissue structure according to a first embodiment of the present invention absorbs a liquid and is recovered to its original state before a compressed tissue is not compressed;

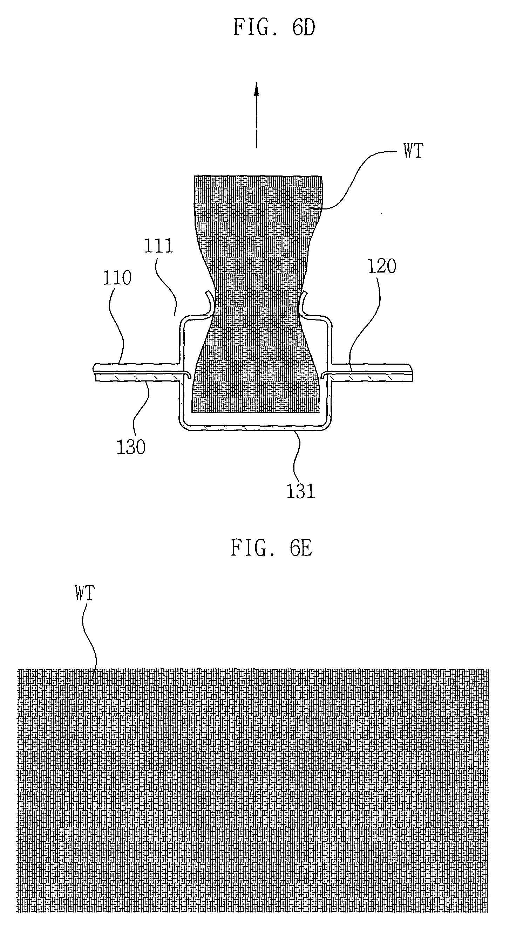

Figure 6D is a cross sectional view illustrating a cylindrical wet tissue which is fabricated in such a manner that a compressed tissue of a disposable tissue structure according to a first embodiment of the present invention absorbs a liquid and is recovered to its original state before a compressed tissue is not compressed; and
Figure 6E is a cross sectional view illustrating a unfolded tissue like a handkerchief for use by a user which is fabricated in such a manner that a compressed tissue of a disposable tissue structure according to a first embodiment of the present invention absorbs a liquid and is recovered to its original state before a compressed tissue is not compressed;

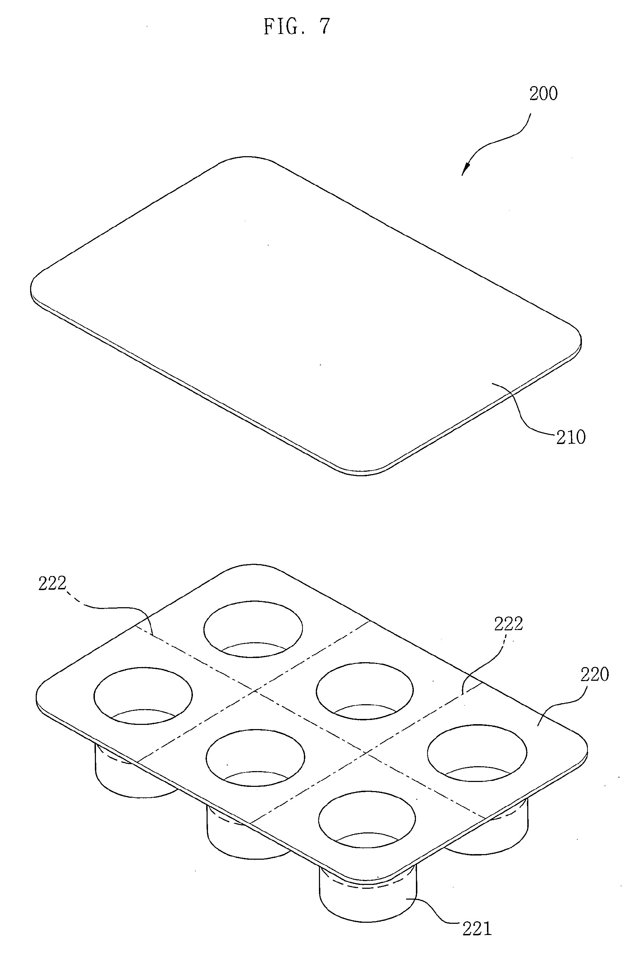

Figure 7 is a disassembled perspective view illustrating a disposable tissue structure according to a second embodiment of the present invention;

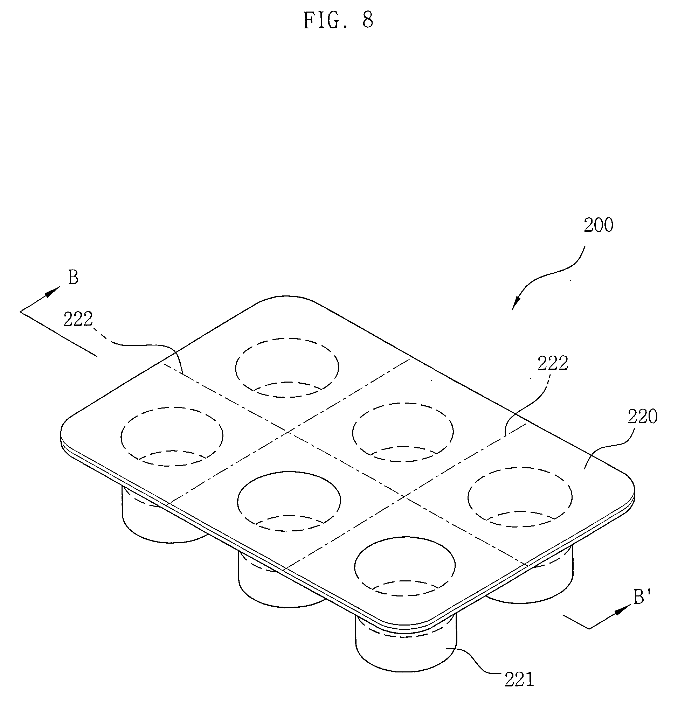

Figure 8 is a perspective view illustrating the construction of a disposable tissue structure according to a second embodiment of the present invention;

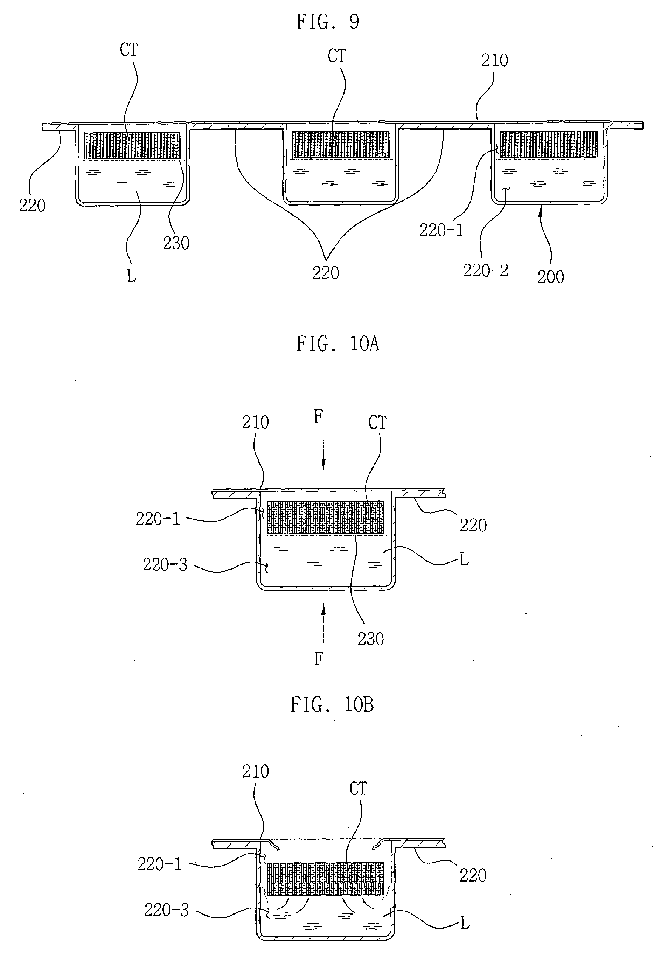

Figure 9 is a cross sectional view taken along line B-B' of Figure 8 for illustrating a disposable tissue structure according to a second embodiment of the present invention;
Figures 10A through 10D are cross sectional views for describing an operation of a disposable tissue structure according to a second embodiment of the present invention, of which:
Figure 10A is a cross sectional view illustrating a state that a certain force is applied from a tissue and liquid storing unit of an upper unit or a lower unit of a disposable tissue structure according to a second embodiment of the present invention;
Figure 10B is a cross sectional view illustrating a state that an upper unit of a disposable tissue structure according to a second embodiment of the present invention is torn when a certain force is applied to the upper side of the upper unit, and a compressed tissue is downwardly moved and contacts with a liquid;

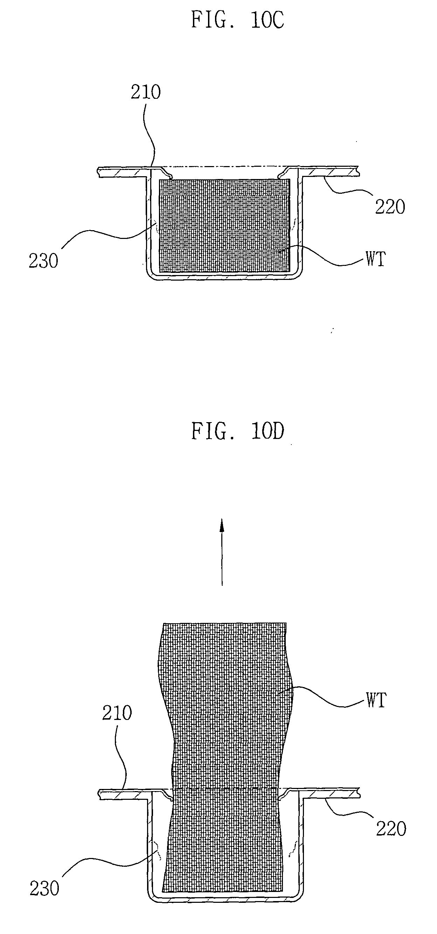

Figure 10C is a cross sectional view illustrating a process that a compressed tissue of a disposable tissue structure according to a second embodiment of the present invention absorbs a liquid and is expanded;
Figure 10D is a cross sectional view illustrating a process that a compressed tissue of a disposable tissue structure according to a second embodiment of the present invention absorbs a liquid and is changed to a cylindrical wet tissue;

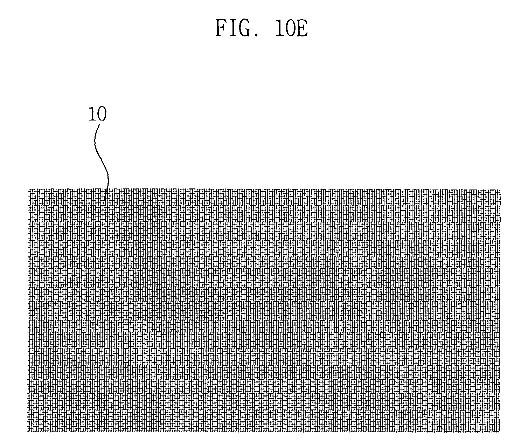

Figure 10E is a view illustrating a unfolded wet tissue which is fabricated in such a manner that a compressed tissue of a disposable tissue structure according to a second embodiment of the present invention absorbs a liquid and is changed to a wet tissue;

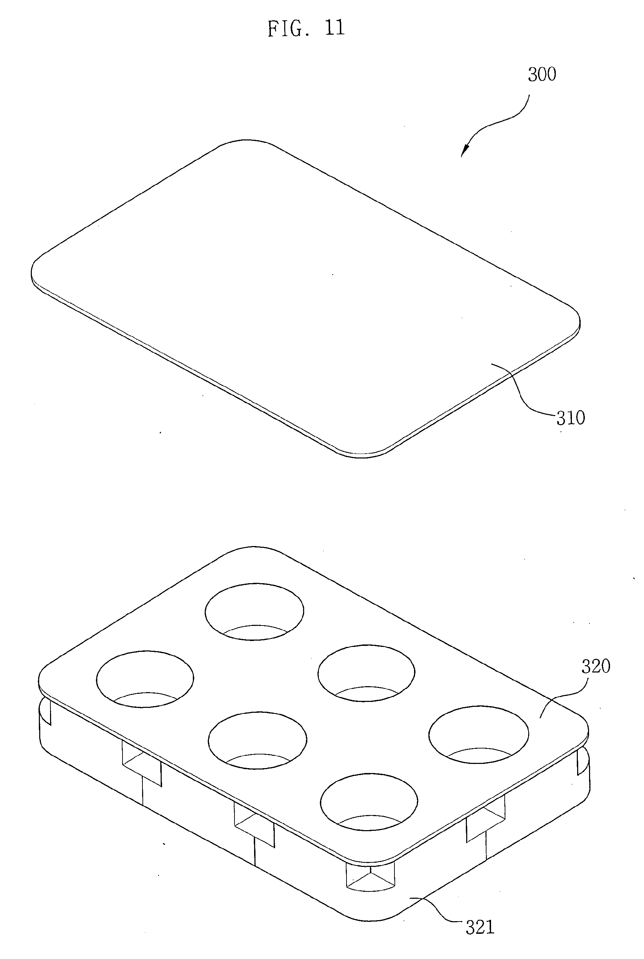

Figure 11 is a disassembled perspective view illustrating the construction of a disposable tissue structure according to a third embodiment of the present invention;

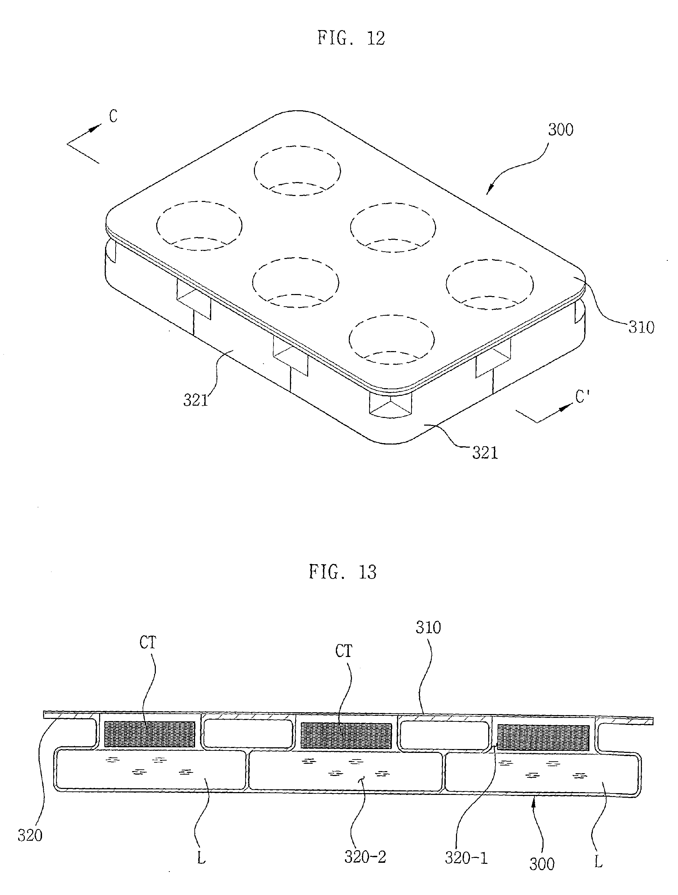

Figure 12 is a perspective view illustrating the construction of a disposable tissue structure according to a third embodiment of the present invention; and
Figure 13 is a cross sectional view taken along liner C-C' of Figure 12 for illustrating the construction of a disposable tissue structure according to a third embodiment of the present invention.

### DETAILED DESCRIPTION OF THE PREFERRED EMBODIMENTS

The preferred embodiments of the present invention will be described with reference to the accompanying drawings.

Referring to Figures 3 and 4, the construction of the disposable tissue structure according to a first embodiment of the present invention will be described.

The disposable tissue structure 100 according to a first embodiment of the present invention includes a certain shaped upper unit 110, a middle unit 120 integrally and sealingly adhered to a lower surface of the upper unit 110, and a lower unit 130 which is integrally and sealingly adhered to a lower surface of the middle unit 120. Here, the upper unit 110, the middle unit 120 and the lower unit 130 are formed of a synthetic resin, pulp, paper or a certain material which is formable. Preferably, the upper unit 110, the middle unit 120 and the lower unit 130 are formed of a synthetic resin. The upper unit 110, the middle unit 120 and the lower unit 130 are sealingly adhered each other. Among the upper unit 110, the middle unit 120, and the lower unit 130, the lower unit 130 has the highest tearing strength. The upper unit 110 and the middle unit 120 may have the same tearing strength. The tearing strength of the upper unit 110 may be higher than that of the middle unit 120.

The construction of the upper unit 110 will be described in detail. The upper unit 110 includes a plurality of hollow cylindrical compressed tissue storing units 111 each of which is upwardly protruded from an upper surface of the upper unit 110 and has an opened lower side and a closed upper side. One compressed tissue(CT) is stored in one compressed tissue storing unit 111. The compressed tissue storing units 111 are formed by a certain number in the vertical and horizontal directions, respectively. In the first embodiment of the present invention, three compressed tissue storing units 111 are formed in the horizontal direction, and two compressed tissue storing units 111 are formed in the vertical direction. Here, the numbers of the rows in the vertical and horizontal directions are not limited. The compressed tissue storing units 111 are cut and separated along a cutting line 112.

The construction of the middle unit 120 sealingly adhered to the lower surface of the upper unit 110 will be described. The middle unit 120 and the upper unit 110 have the same dimension. The middle unit 120 is formed in a flat plate shape having a certain thickness. Therefore, the middle unit 120 has a certain tearing strength and is designed to be torn by a certain external force applied thereto.

The construction of the lower unit 130 sealingly adhered to the lower surface of the middle unit 120 will be described in detail. The lower unit 130 is formed in the same construction as the inverted upper unit 110. The lower unit 130 includes a plurality of hollow cylindrical liquid storing units 131 each of which is integrally downwardly protruded from a lower surface of the lower unit 130 and has an opened upper side and closed lower side. A certain liquid(L) is stored in each of the liquid storing units 131. The liquid storing units 131 are formed by a certain number in the vertical and horizontal directions, respectively. In the first embodiment of the present invention, three liquid storing units 131 are formed in the horizontal direction, and two liquid storing units 131 are formed in the vertical direction. Here, the numbers of the horizontal and vertical direction liquid storing units 131 are not limited. The liquid storing units 131 are separated by a cutting line 132.

Referring to Figure 5, the inner construction of the disposable tissue structure according to a first embodiment of the present invention will be described.

The upper unit 110, the middle unit 120 and the lower unit 130 are stacked and sealingly adhered in sequence. One compressed tissue(CT) is stored in one compressed tissue storing unit 111. A certain liquid having a sterile function, sterilization function, deodorization function, skin caring function, aging prevention function, aroma providing function, etc. or a sterilized water or a distilled water is filled in the liquid storing unit 131. The upper unit 110 and the lower unit 130 are sealingly separated by the middle unit 120 and do not directly contact with each other.

Referring to Figures 6A through 6E, the operation of the disposable tissue structure according to a first embodiment of the present invention will be described.

As shown in Figure 6A, the compressed tissue(CT) is sealingly stored in the interior of the compressed tissue storing unit 110. A certain liquid(L) is sealingly filled in the interior of the liquid storing unit 130. As shown in Figure 6B, when a user applies a certain force(F) using his finger, the upper side of the compressed tissue storing unit 110 is torn and opened, and at the same time the middle unit 120 provided below the compressed tissue(CT) is torn and opened. As shown in Figure 6C, at the time when the middle unit 120 is torn and opened, the liquid(L) stored in the interior of the lower unit 130 is quickly absorbed to the compressed tissue(CT). Therefore, the compressed tissue(CT) is expanded and changed to a cylindrical wet tissue(WT) by absorbing the liquid(L) and is discharged to the outside as shown in Figure 6D. The outwardly discharged cylindrical wet tissue(WT) is unfolded by a user, for thereby being used as a wet tissue as shown in Figure 6E. At this time, the wet tissue(WT) has the same size and construction as the non-woven fabric which is an original form before the non-woven is compressed. Therefore, the user can use a wet tissue from the compressed tissue.

The construction and operation of the disposable tissue structure according to a second embodiment of the present invention will be described with reference to the accompanying drawings.

Referring to Figures 7 and 8, the construction of the disposable tissue structure according to a second embodiment of the present invention will be described.

The disposable tissue structure 200 according to a second embodiment of the present invention includes an upper unit 210 having a flat plate shape, and a lower unit 120 which is sealingly adhered to a lower surface of the upper unit 210. The upper unit 210 and the lower unit 220 may be formed of a synthetic resin, pulp, paper or a certain material. Preferably, the upper unit 210 and the lower unit 220 are formed of a synthetic resin. The upper unit 210 and the lower unit 220 are sealingly adhered each other. Among the upper unit 210 and the lower unit 220, the lower unit 220 has a certain tearing strength higher than that of the upper unit 210.

As shown in Figure 7, the upper unit 210 has the same size, material and construction as the middle unit 120 of the first embodiment of the present invention. Therefore, the detailed description of the same will be omitted.

As shown in Figures 7 and 8, the lower unit 220 has the same construction as the lower unit 130 of the first embodiment of the present invention except for the following construction.

The lower unit 220 of the disposable tissue structure 200 according to a second embodiment of the present invention includes a plurality of hollow cylindrical tissue and liquid storing units 221 each of which is downwardly protruded from a lower surface of the lower unit 220 and has an opened upper side and a closed lower side. Each of the tissue and liquid storing unit 221 is sealingly separated by a middle unit 230. Namely, the interior of each tissue and liquid storing unit 221 is sealingly separated by the middle unit 230 for thereby forming an upper space 220-1 and a lower space 220-2. One compressed tissue(CT) is stored in the upper space 220-1, and a certain liquid(L) is stored in the lower space(220-2). The tissue and liquid storing units 220 are formed by a certain number in the vertical and horizontal directions, respectively. In the second embodiment of the present invention, three tissue and liquid storing units 220 are provided in the horizontal direction, and two tissue and liquid storing units 220 are provided in the vertical direction, respectively. Here, the numbers of the tissue and liquid storing units 220 are not limited. The tissue and liquid storing units 220 are separated by a cutting line 222.

Referring to Figure 9, the inner construction of the disposable tissue structure according to the second embodiment of the present invention will be described.

The upper unit 210 and the lower unit 220 are stacked and adhered sealingly and sequentially. One compressed tissue(CT) is sealingly stored in the upper space 220-1 of the tissue and liquid storing unit 210, and a certain liquid having a sterile function, sterilization function, deodorization function, skin caring function, aging prevention function, aroma providing function, etc. or a sterilized water or a distilled water is filled in the lower space 220-2. The upper space 220-1 and the lower space 220-2 are sealingly separated by the middle unit 230 and do not directly contact with each other.

Referring to Figures 10A through 10D, the operation of the disposable tissue structure according to the second embodiment of the present invention will be described.

As shown in Figure 10A, the compressed tissue(CT) is stored in the upper space 220-1 formed in the interior of the tissue and liquid storing unit 210, and a certain liquid(L) is stored in the lower space 220-2. As shown in Figures 10B and 10C, when a user applies a certain force(F) using his finger, the upper side of the upper unit 210 is torn and opened, and at the same time the middle unit 230 in the interior of the tissue and liquid storing unit 210 is torn and opened. At the time when the middle unit 230 is torn and opened, as shown in Figure 10B, the liquid(L) stored in the lower space 220-2 is quickly absorbed by the compressed tissue(CT). Therefore, the compressed tissue(CT) which absorbed the liquid(L) is changed to a cylindrical wet tissue(WT) and is discharged to the outside as shown in Figure 10D. The outwardly discharged cylindrical wet tissue(WT) is unfolded and used by a user. At this time, the wet tissue(WT) has the same size and construction as the non-woven fabric which is an original material before the non-woven fabric is rolled and compressed. Therefore, the user uses a wet tissue having the same size and construction as the non-woven fabric before it is compressed as shown in Figure 10E.

The construction of the disposable tissue structure according to a third embodiment of the present invention will be described with reference to Figures 11 and 12.

The disposable tissue structure 300 according to a third embodiment of the present invention has the construction similar to the disposable tissue structure 200 of the second embodiment of the present invention. The upper unit 310 of the third embodiment of the present invention is the same as the upper unit 210 of the second embodiment of the present invention. In addition, the lower unit 320 sealingly adhered to the lower surface of the upper unit 210 is the same as the lower unit 220 of the second embodiment of the present invention in their entire construction and inner structure except for the following different construction.

Namely, in the third embodiment of the present invention, the sizes of the upper space 320-1 for storing the compressed tissue(CT) and the lower space 320-2 for storing the liquid(L) which are formed in the interior of the tissue and liquid storing unit 321 of the lower unit 320 are different. Namely, the diameter of the lower space 320-2 is larger than the diameter of the upper space 320-1. Preferably, the height h1 of the upper space 320-1 is higher than the height h2 of the lower space 320-2. In addition, preferably, the heights h1 and h2 of the upper space 320-1 and the lower space 320-2 are same.

Therefore, in the disposable tissue structure according to a third embodiment of the present invention, it is possible to fabricate a thinner disposable tissue in such a manner that the height of the lower space 320-2 for storing the liquid(L) is lower than the height of the lower space 320-2 of the second embodiment of the present invention.

As described above, in the present invention, it is possible to use a disposable wet tissue conveniently anytime anywhere.

In addition, in the present invention, it is possible to easily use a disposable wet tissue in such a manner that a user simply pushes a certain portion of a disposable tissue structure using his finger for thereby fabricating a disposable wet tissue, so that anyone can easily fabricate and use a disposable wet tissue without distinction of age and sex.

It is possible to easily clean a blood remaining during or after menses and a certain secretion from women's sexual organ in such a manner that a disposable wet tissue having a certain liquid having a sterile function, sterilization function, deodorization function, skin caring function, aging prevention function, aroma providing function, etc. is easily fabricated using an improved disposable tissue structure according to the present invention.

It is possible to sanitarily treat a surrounding portion of women's or men's sexual organs because a disposable tissue structure having a certain function according to the present invention is simply and easily carried.

It is possible to easily clean a dirty face, hands, etc. during tour.

It is possible to implement a healthy sexual life by sanitarily treating a women's and men's sex organ after a sexual intercourse between married couple.

It is possible to sanitarily treat a secretion of baby or infant after piss and feces during a tour with baby and infant.

It is possible to sanitarily clean a user's hands before meal at home or in the restaurants because it is possible to easily carry a disposable tissue.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. A disposable tissue structure, comprising:
an upper unit having a plurality of compressed tissue storing units, each compressed tissue storing unit having a lower opened side and an upper closed side and being hollow and upwardly protruded from an upper surface of the upper unit for storing a compressed tissue therein which is compressed using a non-woven fabric, pulp or the like and is formed in a certain shape, so that the compressed tissue is changed to a disposable wet tissue when a certain liquid is absorbed to the compressed tissue;
a middle unit which is sealingly adhered to a lower surface of the upper unit and has a certain thickness and tearing strength, said middle unit being forked in a flat plate shape; and
a lower unit which is sealingly adhered to a lower surface of the middle unit and has a plurality of liquid storing units which are formed in the same construction as the inverted upper unit, said liquid storing unit having an opened upper side and closed lower side and being hollow and downwardly protruded from a lower surface of the lower unit for thereby storing a liquid having a certain function.

2. The structure of claim 1, wherein said upper unit, middle unit and lower unit are formed of the same material, preferably, are formed of a synthetic resin.

3. The structure of claim 1, wherein said upper unit, middle unit and lower unit have the same dimension.

4. The structure of claim 1, wherein said upper unit, middle unit and lower unit each have a different thickness.

5. The structure of claim 1, wherein said upper unit, middle unit and lower unit each have a different tearing strength.

6. The structure of claim 1, further comprising a cutting line between a plurality of the compressed tissue storing units and a plurality of the liquid storing units, respectively.

7. The structure of claim 1, wherein the tearing strength of the lower unit is higher than that of the upper unit or the middle unit.

8. The structure of claim 1, wherein the tearing strengths of the upper unit and the middle unit are same.

9. The structure of claim 1, wherein a liquid having a certain function such as a sterile function, sterilization function, deodorization function, skin caring function, aging prevention function, aroma providing function, surface active agent function, etc. is filled in each liquid storing unit.

10. The structure of claim 1, wherein a plurality of compressed tissue storing units upwardly protruded from an upper surface of the upper unit and distanced from each other are provided by a certain number in a horizontal direction and vertical direction, respectively.

11. The structure of claim 1, wherein a plurality of liquid tissue storing units downwardly protruded from a lower surface of the lower unit and distanced from each other are provided by a certain number in a horizontal direction and vertical direction, respectively.

12. The structure of claim 1, wherein when a plurality of the compressed tissue storing units and a plurality of the liquid storing units are assembled with each other, the compressed tissue storing units and the liquid storing units correspond with each other.

13. A disposable tissue structure, comprising:
a flat plate shaped upper unit which has a certain thickness and a tearing strength; and
a lower unit which is sealingly adhered to a lower surface of the upper unit and has a plurality of tissue and liquid storing units, each of said tissue and liquid storing units having an opened upper side and a closed lower side and being downwardly protruded in a certain shape for storing a compressed tissue therein which is compressed using a non-woven fabric, pulp or the like and is formed in a certain shape, so that the compressed tissue is changed to a disposable wet tissue when a certain liquid is absorbed to the compressed tissue, and each of said tissue and liquid storing units being capable of storing a certain liquid therein for thereby fabricating a disposable wet tissue using a compressed tissue.

14. The structure of claim 13, wherein said upper unit and said lower unit are formed in the same material, preferably, are formed of a synthetic resin.

15. The structure of claim 13, wherein said upper unit and said lower unit each have a different thickness.

16. The structure of claim 13, wherein said upper unit and said lower unit each have a different tearing strength.

17. The structure of claim 13, wherein the interior of each tissue and liquid storing unit is sealingly divided into an upper space and a lower space by a middle unit, respectively.

18. The structure of claim 14, wherein the volume of the lower space is larger than the volume of the upper space.

19. The structure of claim 14, wherein the height of the lower space is lower than that of the upper space.

20. The structure of claim 14, wherein the diameter of the lower space is larger than the diameter of the upper space.

21. The structure of claim 14, wherein a compressed tissue which is compressed using a non-woven fabric, pulp or the like and is formed in a certain shape, so that the compressed tissue is changed to a disposable wet tissue when a certain liquid is absorbed to the compressed tissue, is stored in the upper space formed in the lower unit.

22. The structure of claim 14, wherein a certain functional liquid having a sterile function, sterilization function, deodorization function, skin caring function, aging prevention function, aroma providing function, surface active agent function, etc. is filled in the lower space formed in the lower unit.

23. The structure of claim 13, further comprising a cutting line between a plurality of the tissue and liquid storing units, respectively.

24. The structure of claim 13, wherein a tearing strength of the lower layer is higher than that of the upper unit or the middle unit.

25. The structure of claim 13, wherein a tearing strength of the upper unit is the same as that of the middle unit.

26. The structure of claim 13, wherein a plurality of tissue and liquid storing units downwardly protruded from a lower surface of the lower unit and distanced from each other are provided by a certain number in a horizontal direction and vertical direction, respectively.
